# FASCICULE DE BREVET EUROPEEN

(11) **EP 1 870 042 B1**
(45) Date de publication et mention de la délivrance du brevet: **29.07.2009**
(21) Numéro de dépôt: 07110792.4
(22) Date de dépôt: 21.06.2007
(51) Int. Cl.: A61B 17/064, A61B 17/68, A61B 17/068, A61B 17/00

(54) **Ensemble chirurgical de pose d'agrafe de compression**
Chirurgisches Kit zum Einsetzen von Kompressionsklammern
Surgical assembly for applying a compression staple

(30) Priorité: 21.06.2006 FR 0605548
(43) Date de publication de la demande: 26.12.2007
(73) Titulaire: Textile Hi-Tec (T.H.T.), 34220 Verreries de Moussans (FR)
(72) Inventeur: Houard, William, 81270, LABASTIDE ROUAIROUX (FR)
(74) Mandataire: Hennion, Jean-Claude

(56) Documents cités:
- FR-A- 2 874 166
- US-A- 5 788 698
- US-A- 5 853 414
- US-A1- 2005 096 660

## Description

La présente invention concerne le domaine des instruments chirurgicaux. Elle concerne plus particulièrement un ensemble chirurgical spécialement destiné à la pose d'agrafe en vue de réaliser la compression d'une partie osseuse dans le cadre d'une ostéosynthèse.

Pour faciliter la réparation d'une fracture, l'ostéosynthèse consiste à réunir les fragments de l'os fracturé à l'aide d'un matériel permettant d'immobiliser le foyer de fracture pendant un temps nécessaire à la consolidation. Il peut s'agir de plaques, de vis ou encore d'agrafes.

Une agrafe d'ostéosynthèse ou agrafe de compression comprend deux pattes ou branches destinées à pénétrer dans les parties osseuses à réunir et une partie centrale ou pont. Pour qu'une telle agrafe soit réellement efficace, il faut qu'après la pose des deux pattes dans les trous percés dans les parties osseuses il s'exerce une force visant à rapprocher tout ou partie des deux pattes l'une de l'autre, permettant d'appliquer les deux surfaces osseuses au niveau du foyer de fracture l'une contre l'autre avec une certaine pression.

On a déjà proposé un grand nombre de structures d'agrafes permettant d'atteindre cet objectif. Dans le document FR 2 743 490, l'effet de compression est obtenu sans donner à l'agrafe une forme particulière au niveau du pont mais par le choix du matériau dont elle est formée, étant réalisée en titane T40 ou autre matériau de titane ou à base de titane malléable à froid, présentant une résistance minimale à la traction de 345 Mpa, une limite minimale conventionnelle d'élasticité de 230 Mpa et un allongement de 20 %.

Le document US 2005/0096660 décrit une agrafe de compression en forme de U munie de deux pattes, réunie par un pont élastique et courbé.

Dans le document CA 1 149 106, l'agrafe se caractérise en ce que le pont présente des moyens pour obtenir, après la pose de l'agrafe, une déformation permanente raccourcissant la longueur du pont. C'est ce raccourcissement de la longueur du pont qui permet d'exercer la force de compression entre les deux pattes.

Dans le document SU 1 463 268, les pattes de l'agrafe sont convergentes et le pont est courbé en arc de cercle dont le centre est le pont de convergence des pattes.

Comme souligné dans le document EP 0 695 142, la pose d'une telle agrafe est extrêmement délicate car les branches doivent être écartées élastiquement pour être logées dans des alésages préalablement percés à un angle inférieur à l'angle des branches.

C'est le but de la présente invention que de pallier cette difficulté, pour la pose d'une agrafe de compression ayant sensiblement la structure de l'agrafe décrite dans le document SU 1 463 268.

Il s'agit d'un ensemble chirurgical de pose d'agrafe de compression qui comprend une agrafe avec deux pattes longitudinales reliées par un pont transversal et un ancillaire de pose.

De manière caractéristique, l'agrafe est dans un matériau à mémoire de forme, ayant au repos lesdites pattes qui sont convergentes d'un angle α, et en configuration active d'implantation dans l'os, lesdites pattes qui sont soit parallèles soit qui ont un angle de convergence β inférieur à α.

De plus, l'ancillaire comporte :
a) une ouverture distale débouchant sur une cavité interne qui sert de logement pour l'agrafe au repos, dont les pattes sont tournées vers ladite ouverture et,
b) une pièce d'écartement, actionnable en déplacement selon un premier mouvement vertical dans lequel la pièce d'écartement vient en contact avec les faces internes des deux pattes et applique sur celles-ci une force réalisant leur écartement jusqu'à la configuration active d'implantation et
c) des moyens d'entraînement de l'agrafe en configuration active, incluant la pièce d'écartement, selon un mouvement horizontal permettant la sortie de l'agrafe hors de son logement par l'ouverture distale.

Ainsi, il suffit au praticien d'introduire les deux pattes longitudinales de l'agrafe en configuration active d'implantation dans les alésages préalablement percés dans l'os puis de déplacer la pièce d'écartement selon un second mouvement vertical en sens inverse du premier pour libérer l'agrafe de la pièce d'écartement. Dans cette position, on comprend qu'il subsiste entre le pont transversal et l'os un espace libre qui correspond au minimum à l'espace qui était précédemment occupé par la pièce d'écartement. Il suffit alors d'exercer une pression sur le pont pour que la pose de l'agrafe soit complète.

Dans une variante de réalisation, la pièce d'écartement est une plaque dont l'extrémité a une forme générale de trapèze isocèle, la petite base dudit trapèze correspondant au côté d'introduction de la pièce d'écartement dans l'espace interne entre les deux pattes de l'agrafe au repos, en avant du pont transversal. Les faces latérales divergentes dudit trapèze isocèle sont conformées en sorte de s'appliquer sur les faces internes des deux pattes de l'agrafe et de repousser celles-ci, lors du premier mouvement vertical, jusqu'à ce que les dites pattes soient dans la configuration active d'implantation dans l'os.

Selon une variante de réalisation, les moyens d'entraînement consistent en un poussoir, à l'avant et sous lequel est montée la pièce d'écartement, ledit poussoir comportant une tête supérieure de manoeuvre, une tête inférieure, une tige verticale de liaison entre les deux dites têtes. De plus, l'ancillaire comporte au droit de la cavité interne, servant de logement à l'agrafe, une première ouverture débouchant sur sa face supérieure et une seconde ouverture débouchant sur sa face inférieure. Enfin, le poussoir est monté sur l'ancillaire avec la tige de liaison qui passe à travers la cavité interne et les deux dites ouvertures et avec les têtes supérieure et inférieure qui font office de butées respectivement contre la face supérieure et contre la face inférieure de l'ancillaire, de part et d'autre desdites ouvertures, lors du mouvement horizontal du poussoir.

Selon un premier mode de réalisation, la partie distale de l'ancillaire est composée de deux bras latéraux globalement parallèles, s'étendant de part et d'autre de la cavité interne et des deux ouvertures supérieure et inférieure et par deux montants transversaux s'étendant entre les deux dits bras latéraux de part et d'autre de l'ouverture distale. Le poussoir vient en butée, en fin de son mouvement horizontal, contre les deux montants transversaux et le mouvement vertical de libération de l'agrafe intervient alors que le poussoir est encore solidaire de l'ancillaire. Dans ce cas, l'espace libre qui subsiste entre le pont transversal et l'os, après libération de l'agrafe, correspond à l'espace qui était précédemment occupé d'une part par la pièce d'écartement et d'autre part par les montants transversaux.

Par ailleurs, dans ce mode de réalisation, dans sa position au repos, à l'intérieur de la cavité interne, les extrémités libres des deux pattes de l'agrafe reposent sur les deux montants transversaux, lesquels s'étendent entre les deux bras latéraux de part et d'autre de l'ouverture distale. Du fait de cette position relativement instable, il est souhaitable que le praticien ait à disposition un ancillaire pré monté, avec l'agrafe disposée dans la cavité interne qui lui sert de logement et la pièce d'écartement déjà pré engagée pour que l'agrafe soit bloquée en position par ladite pièce d'écartement et ne puisse s'échapper de l'ancillaire par l'ouverture distale.

Dans un autre mode de réalisation, préféré par rapport à celui décrit ci-dessus, la partie distale de l'ancillaire est délimitée par deux bras latéraux flexibles, s'étendant de part et d'autre de la cavité interne et des deux ouvertures supérieure et inférieure , les deux dits bras étant convergents au repos et étant aptes à s'écarter l'un de l'autre jusqu'à devenir sensiblement parallèles lors du mouvement horizontal du poussoir du fait qu'ils sont repoussés par la pièce d'écartement et éventuellement la tige de liaison.

Selon cette disposition, les deux pattes convergentes au repos de l'agrafe sont en appui dans la partie de cavité interne qui est formée dans les deux bras latéraux flexibles convergents, ce qui améliore la stabilité du positionnement de l'agrafe à l'intérieur de ladite cavité.

De préférence, dans ce mode de réalisation, la partie distale de l'ancillaire est exempte de montants transversaux de sorte que le poussoir peut, en fin du mouvement horizontal, se détacher de l'ancillaire avec l'agrafe en configuration active d'implantation dans l'os. De ce fait l'espace libre qui subsiste entre le pont transversal et l'os correspond strictement à l'espace précédemment occupé par la pièce d'écartement.

Afin de faciliter le travail du praticien, l'ancillaire de la présente invention peut comporter, vers sa partie proximale, deux guides tubulaires de perçage dont l'entraxe et le positionnement angulaire correspondent à ceux des pattes de l'agrafe en configuration active. L'ancillaire peut également comporter, vers sa partie proximale, un foret de perçage dont le diamètre correspond à l'alésage nécessaire à l'implantation des pattes de l'agrafe. Ainsi, le praticien a à sa disposition, dans le même ensemble chirurgical, tous les éléments qui lui sont nécessaires pour réaliser l'implantation de l'agrafe de compression.

Avantageusement l'extrémité proximale de l'ancillaire comporte une rainure transversale qui est configurée pour recevoir de manière sensiblement ajustée le pont de l'agrafe, après implantation de celle-ci dans l'os, en vue de finaliser son impaction. Il suffit au praticien, après avoir introduit les pattes de l'agrafe dans les alésages et dégagé le poussoir ou l'ancillaire, de retourner ce dernier, de telle sorte que le pont de l'agrafe vienne s'encastrer dans la rainure transversale et d'appliquer une certaine pression sur l'ancillaire pour finaliser l'impaction de l'agrafe.

L'agrafe peut de manière conventionnelle comporter des ergots, anti-migration, qui peuvent être soit sur les faces externes des deux pattes soit sur les faces internes. Dans ce dernier cas, on comprend que les ergots anti-migration ne peuvent pas se trouver sur la zone d'application de la pièce d'écartement, à proximité du pont transversal, puisqu'ils constitueraient une gêne pour la pénétration de ladite pièce d'écartement lors de son mouvement vertical.

Avantageusement, l'agrafe est dans au moins un matériau biodégradable et/ou résorbable, stérilisable, d'origine naturelle ou synthétique, notamment choisi dans le groupe :
- polylactide, polyglycolide, poly-ecaprolactone,
- polyhydroxy butyrate, polyhydroxy valérate,
- poly carbonates et assimilés,
- cellulose, polysaccharides, amidon.

De préférence, dans ce cas, le matériau dans lequel est réalisée l'agrafe est chargé de poudre et/ou de nano poudre d'hydroxy-apatite et/ou de phosphate de calcium, notamment de β tricalcium phosphate, et/ou d'hydrogéno phosphate de calcium et/ou de carbonate de calcium et/ou d'hydrogéno carbonate de calcium, de préférence à raison de 5 à 60% en poids de charge.

La présente invention sera mieux comprise à la lecture de la description qui va être faite de deux exemples de réalisation d'un ensemble chirurgical de pose d'une agrafe de compression comprenant deux pattes longitudinales , à implanter dans l'os reliées à un pont transversal, illustrés par le dessin annexé dans lequel :
La figure 1 est une vue en perspective de dessus d'un premier exemple d'ensemble chirurgical dont l'ouverture distale est délimitée par deux bras latéraux et par deux montants transversaux,
La figure 2 est une représentation en perspective du poussoir et de la pièce d'écartement du premier exemple, en position de pré chargement,
La figure 3 est une représentation schématique en perspective de l'ensemble chirurgical de la figure 1 en fin de mouvement vertical de la pièce d'écartement,
La figure 4 est une vue schématique en perspective de la pièce d'écartement et du poussoir du premier exemple, avec l'agrafe en configuration active d'implantation dans l'os,
La figure 5 est une représentation schématique en perspective de l'ensemble chirurgical des figures 1 et 3 avec le poussoir en fin de mouvement horizontal, l'agrafe étant en position active d'implantation dans l'os,
La figure 6 est une vue schématique partielle en perspective de l'ensemble de la figure 5 après implantation de l'agrafe dans l'os et dégagement de l'ancillaire.
La figure 7 est une vue schématique partielle en perspective de l'extrémité proximale de l'ancillaire, avec les deux guides tubulaires de perçage et le foret de perçage,
La figure 8 est une vue schématique partielle en perspective d'un second exemple de réalisation dans lequel l'ouverture distale de l'ancillaire est délimitée par deux bras latéraux flexibles, l'agrafe étant en position de repos,
La figure 9 est une vue schématique partielle en perspective de l'ensemble chirurgical de la figure 8 avec le bouton-poussoir qui s'est déplacé selon le mouvement horizontal avec les deux pattes de l'agrafe sorties de l'ouverture distale et
La figure 10 est une vue schématique partielle en perspective de l'ensemble chirurgical des figures 8 et 9 après la sortie du poussoir de l'ancillaire et avant libération de l'agrafe.

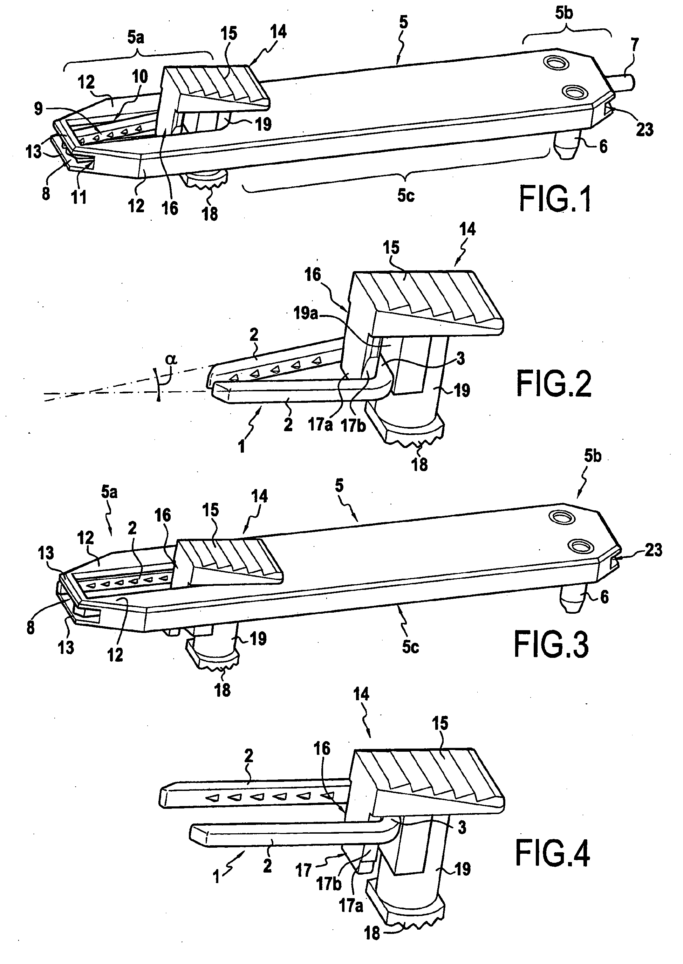

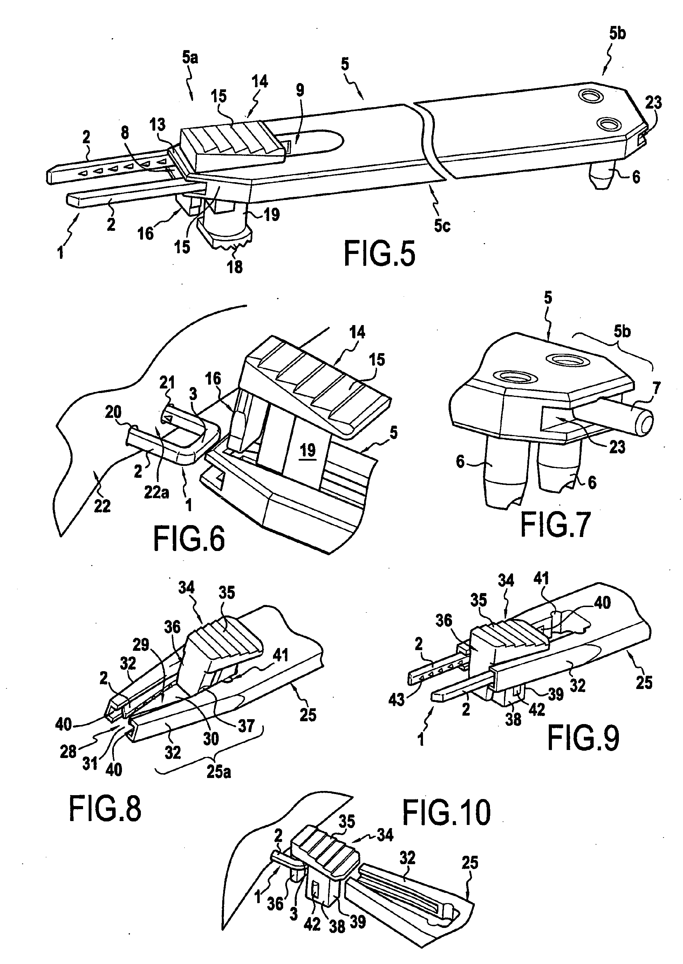

L'ensemble chirurgical visé par la présente invention concerne la pose d'une agrafe 1 d'ostéosynthèse, dénommée également agrafe de compression qui est visible sur la figure 2 dans sa configuration au repos et sur les figures 4 et 6 dans sa configuration active d'implantation dans l'os.

Cette agrafe comprend deux pattes longitudinales 2 qui sont reliées à leurs extrémités par un pont transversal 3.

Dans la configuration au repos (figure 2), les deux pattes rectilignes 2 sont convergentes, faisant entre elles un angle α de l'ordre de 7 à 15°. Dans la configuration active d'implantation dans l'os (figures 4 et 6), les deux pattes 2 sont sensiblement parallèles ou sont convergentes avec un angle β qui est inférieur à l'angle α. L'agrafe 1 est réalisée dans un matériau à mémoire de forme de telle sorte que lorsque l'agrafe est dans sa configuration active d'implantation dans l'os, les deux pattes ont naturellement tendance à retrouver leur configuration au repos, exerçant de ce fait une force de compression vis-à-vis de la partie osseuse qui se trouve disposée entre elles. C'est cette force de compression qui est recherchée lors de l'ostéosynthèse.

Dans le premier exemple de réalisation qui est illustré aux figures 1 à 6, l'ensemble chirurgical 4 comprend, en plus de l'agrafe 1, un ancillaire de pose 5 qui se présente globalement comme une plaque de section rectangulaire dont la partie distale 5a sert à la pose de l'agrafe 1, dont la partie proximale 5b comporte des éléments additionnels tels que deux guides tubulaires de perçage 6 et un foret de perçage 7, utiles au praticien, et dont la partie intermédiaire 5c sert de poignée de préhension lors de la manipulation de l'ancillaire 5 par le praticien.

La partie distale 5a de l'ancillaire 5 comporte une ouverture distale 8 destinée à l'introduction et à l'éjection de l'agrafe 1, laquelle se trouve logée dans une cavité interne 9, à l'intérieur de ladite partie distale 5a. Cette cavité 9 est prévue pour accueillir l'agrafe 1 dans sa configuration au repos, avec ses deux pattes en position convergente.

L'ancillaire 5 comporte, au droit de ladite cavité interne 9, une première ouverture centrale supérieure 10 débouchant sur la face supérieure de l'ancillaire et une seconde ouverture centrale inférieure 11 débouchant sur la face inférieure de l'ancillaire. Ainsi, la partie distale 5a de l'ancillaire est constituée d'une part par deux bras latéraux 12 globalement parallèles, s'étendant de part et d'autre de la cavité interne 9 et des deux ouvertures centrales supérieure 10 et inférieure 11 et d'autre part par deux montants transversaux 13 qui s'étendent entre les deux dits bras latéraux 12 de part et d'autre de l'ouverture distale 8.

Dans sa position de repos, l'agrafe 1 se trouve vers l'avant de la cavité interne 9, avec les deux extrémités de ses pattes 2 qui reposent sur le montant transversal inférieur 13. A l'opposé, les portions latérales de l'agrafe 1, au niveau du pont transversal 3, reposent dans la cavité interne 9, ladite cavité se prolongeant latéralement par une rainure dans les montants latéraux 12, de part et d'autre des ouvertures centrales 10, 11.

L'ancillaire 5 comporte une pièce d'écartement 16 qui, dans l'exemple illustré aux figures, est intégrée dans un bouton-poussoir 14 qui permet de déplacer ladite pièce d'écartement 16 selon deux mouvements à savoir un mouvement vertical et un mouvement horizontal. Plus précisément, le bouton-poussoir 14 comporte une tête supérieure 15, à l'avant et sous laquelle est fixée la pièce d'écartement 16. Celle-ci consiste en une plaque dont la partie inférieure 17 a la forme générale d'un trapèze isocèle, dont la petite base 17a correspond au côté d'introduction de la pièce d'écartement 16 dans l'espace interne entre les deux pattes 2 de l'agrafe au repos, en avant du pont transversal 3.

Le bouton-poussoir 14 comporte une tête inférieure 18 qui est reliée à la tête supérieure de manoeuvre 15 par une tige verticale de liaison 19. Le bouton-poussoir 14 est monté sur l'ancillaire 5 avec la tige de liaison 19 qui passe à travers la cavité interne 9 et les deux ouvertures supérieure 10 et inférieure 11. Les têtes supérieure 15 et inférieure 18 du bouton-poussoir 14 ont chacune une largeur supérieure à celle des ouvertures 10, 11 de telle manière que lesdites têtes supérieure 15 et inférieure 18 font office de butées respectivement contre la face supérieure et la face inférieure de l'ancillaire, de part et d'autre desdites ouvertures 10, 11.

On a représenté sur les figures 1 et 2 le bouton-poussoir 14 dans sa position de blocage initial de l'agrafe 2 dans sa configuration au repos, après introduction de celle-ci dans la cavité interne 9 par l'ouverture distale 8. La cavité interne 9 ainsi que les deux ouvertures supérieure 10 et inférieure 11 sont surdimensionnées, quant à leur longueur, par rapport à la longueur de l'agrafe 2 en sorte de permettre le placement à l'arrière de ladite cavité interne 9 de la tige de liaison 19, le pont transversal 3 venant en appui contre la face avant 19a de ladite tige de liaison 19. Dans cette première position, le bouton-poussoir 14 a été légèrement abaissé verticalement de telle sorte que la partie d'extrémité 17a, correspondant à la petite base du trapèze isocèle 17 formé par la pièce d'écartement 16, pénètre dans l'espace délimité d'une part par le pont transversal 3 et d'autre part par les deux pattes 2. Ladite pièce d'écartement 16 est à une distance de la face avant 19a de la tige de liaison 19 qui est juste légèrement supérieure à l'épaisseur du pont transversal 3 de l'agrafe 2. Dans cette position, l'agrafe 2 est bloquée à l'intérieur de la cavité mais reste encore en position de repos avec ses deux pattes 2 convergentes.

C'est dans cette position que l'ensemble chirurgical 4 est mis à la disposition du praticien. Celui-ci commence par percer, dans la partie osseuse, les deux alésages 20, 21 dont il a besoin pour réaliser l'ostéosynthèse. Pour ce faire, il a à sa disposition le foret de perçage 7 dont le diamètre correspond aux dimensions des pattes 2 de l'agrafe 1, foret qui est emmanché dans un trou pratiqué dans la partie proximale 5b de l'ancillaire. Pour percer ces alésages le praticien utilise les deux guides de forage 6 qui équipent également la partie proximale 5b de l'ancillaire 5. Il s'agit de deux tubes creux dont l'évidement intérieur traverse totalement l'ancillaire 5. L'écartement et l'orientation des deux guides 6 sont déterminés pour que les alésages 20, 21 permettent la mise en place de l'agrafe 2 dans sa configuration active d'implantation dans l'os, telle qu'illustrée aux figures 4 et 6. Les deux guides peuvent avoir des direction strictement parallèles ou éventuellement convergentes, avec entre elles un angle β qui doit, dans ce cas, être inférieur à l'angle α entre les deux pattes 2 dans leur configuration au repos.

Le praticien peut alors se saisir de l'ancillaire 5 par sa partie intermédiaire 5c, déplacer le bouton-poussoir 14 dans un mouvement vertical, en appuyant sur la tête supérieure de manoeuvre 15, ce qui entraîne corrélativement le mouvement vertical de la pièce d'écartement 16. Lors de ce mouvement, les faces latérales divergentes 17b du trapèze isocèle s'appuient contre les faces internes des pattes latérales 2, les repoussant l'une de l'autre jusqu'à ce que celles-ci adoptent la configuration active d'implantation dans l'os à l'issue de ce mouvement vertical de haut en bas. C'est cette configuration qui est illustrée aux figures 3 et 4, la tête supérieure de manoeuvre 15 venant alors en butée sur la face supérieure de l'ancillaire.

Le praticien peut alors déplacer le bouton-poussoir 14 vers l'avant en poussant la tête supérieure de manoeuvre 15. Ce mouvement horizontal entraîne corrélativement la sortie des pattes 2 de l'agrafe 1 par l'ouverture distale 8 de l'ancillaire. Le praticien fait pénétrer lesdites pattes 2 dans les alésages 20, 21 qu'il a précédemment percés dans la partie osseuse. Etant donné que les alésages sont percés avec l'écartement et l'orientation qui correspondent aux configurations actives de l'agrafe 1, cette introduction ne pose pas de difficulté particulière.

Lorsque la pièce d'écartement 16 est en butée contre les montants transversaux 13 de la partie distale 5a de l'ancillaire 5 et que les pattes 2 de l'agrafe 1 ont été introduites au maximum dans les alésages 20, 21, le praticien peut déplacer le bouton-poussoir 14 dans un second mouvement vertical, inverse du premier soit en tirant sur la tête supérieure 15 soit en poussant sur la tête inférieure 18. Ce second mouvement vertical permet de dégager la pièce d'écartement 16 de l'agrafe 1, désolidarisant l'ancillaire 5 de ladite agrafe 1. C'est cette position qui est illustrée à la figure 6, avec l'agrafe 1 dont les pattes sont en configuration active dans les alésages 20, 21 sensiblement parallèles et avec l'ancillaire 5 qui est en retrait par rapport au pont transversal 3 de l'agrafe 1.

Dans cette position, la pose de l'agrafe 1 n'est pas terminée puisqu'il subsiste entre le pont transversal 3 et la face extérieure 22a de la partie osseuse 22, un espace qui correspond d'une part à la largeur des montants transversaux 13 de la partie distale 5a de l'ancillaire 5 et d'autre part à la largeur de la pièce d'écartement 16. Pour finaliser la pose de l'agrafe 1, il suffit d'impacter l'agrafe au niveau du pont transversal 3 soit par une simple pression manuelle soit par une pression communiquée en utilisant la partie proximale 5b de l'ancillaire 5. Comme illustré à la figure 7, cette partie proximale comporte une rainure transversale 23 qui est configurée pour recevoir de manière sensiblement ajustée le pont transversal 3 de l'agrafe 1. Bien sûr lors de cette impaction, le foret 7 qui a été utilisé lors du forage des alésages, est retiré.

Dans le second exemple de réalisation, l'ancillaire 25 diffère de l'ancillaire 5 du premier exemple par la structure particulière de sa partie distale 25a.

Celle-ci est constituée de deux bras latéraux flexibles 32 qui s'étendent de part et d'autre de la cavité interne 29 et des deux ouvertures supérieure 30 et 31. Lorsque l'agrafe 1 est insérée à l'intérieur de la cavité interne 29, dans sa configuration au repos, les deux bras flexibles 32 sont également convergents (figure 8). Ainsi, contrairement au premier mode de réalisation, l'ancillaire 25 ne comporte pas, de part et d'autre de son ouverture distale 28 de montants transversaux.

Dans sa configuration au repos, l'agrafe 1 a ses deux pattes convergentes 2 qui sont en appui à l'intérieur de la cavité interne 29 dans deux rainures 40 latérales creusées dans les deux bras latéraux flexibles 32.

Les ouvertures supérieure 30 et inférieure 31 formées à l'aplomb de la cavité interne 29 et débouchant respectivement sur la face supérieure et sur la face inférieure de l'ancillaire 25 se prolongent jusqu'à l'ouverture distale 28.

Le bouton-poussoir 34 de ce second exemple a globalement la même structure que celui 14 du premier exemple, avec sa pièce d'écartement 36 dont l'extrémité 37 a une forme en trapèze isocèle, avec sa tête supérieure 35 de manoeuvre, sa tête inférieure 38 et la tige de liaison 39.

A la différence du bouton-poussoir 14 du premier exemple, la tête inférieure 38 a la même section transversale que la tige de liaison 39 exception faite de deux épaulements latéraux 42. La cavité interne 29 et les deux ouvertures supérieure 30 et inférieure 31 sont conformées, en arrière des rainures latérales 40 prévues pour servir de logements aux pattes 2 de l'agrafe 1, pour permettre le coulissement vertical de la tête inférieure 38 et de la tige de liaison 39 de haut vers le bas. La tête supérieure de manoeuvre 35 vient, en fin de ce déplacement vertical, en butée sur la face supérieure de l'ancillaire 25. On a donc deux encoches verticales 41 qui correspondent aux épaulements 42 de la tête inférieure 38 et qui permettent ce coulissement.

Dans ce second exemple de réalisation, le bouton-poussoir 34 est indépendant de l'ancillaire 25.

Pour le chargement de l'agrafe 1, on introduit celle-ci par l'ouverture distale 28 jusqu'à ce que le pont transversal 3 vienne en butée au fond des rainures 40. On introduit ensuite le bouton-poussoir 34 depuis la face supérieure de l'ancillaire 25 en introduisant la tête inférieure 38 dans l'ouverture supérieure 30 et en faisant coulisser le bouton-poussoir 34 vers le bas, les épaulements 42 étant guidés par les encoches verticales 41. Lors de ce déplacement vertical, la pièce d'écartement 36 est venue s'insérer en avant du pont transversal 3 entre les deux pattes 2 de l'agrafe 1. Le déplacement vertical peut être limité en cas de pré chargement de l'agrafe, celle-ci restant en configuration au repos comme illustré à la figure 8. On réalise le passage de la configuration au repos à la configuration active d'implantation en terminant le déplacement vertical jusqu'à écartement des deux pattes 2 de l'agrafe 1 et des deux bras flexibles 32, puis on déplace le bouton-poussoir 34 vers l'avant comme illustré à la figure 9. Le bouton-poussoir est parfaitement maintenu en ligne grâce aux deux butées constituées d'une part par la tête supérieure de manoeuvre 35 et d'autre part par les épaulements 42 de la tête inférieure 38, lesdits épaulements venant en appui sur la face inférieure de l'ancillaire 25.

Dans la configuration de la figure 9, le praticien a procédé à l'introduction des deux pattes 2 de l'agrafe 1 dans les alésages préalablement percés dans la partie osseuse. Il peut alors procéder dans un premier temps au dégagement de l'ancillaire 25 (figure 10) et dans un second temps du bouton-poussoir 34. Pour cela il lui suffit de tirer sur la tête supérieure de manoeuvre 35 ou de pousser sous la tête inférieure 38, afin de dégager la pièce d'écartement 36 des pattes 2 de l'agrafe 1.

Il lui suffit ensuite, comme dans le premier exemple de réalisation, de finaliser l'impaction de l'agrafe 1 soit manuellement soit à l'aide de la rainure transversale se trouvant dans la partie proximale de l'ancillaire.

L'agrafe 1 peut comporter, comme illustré, des ergots 43 anti migration, ces ergots peuvent être disposés sur les faces externe ou interne des deux pattes latérales 2. Cependant, lorsqu'ils sont sur les faces internes, ils ne peuvent se trouver sur la zone d'application de la pièce d'écartement, immédiatement à proximité du pont transversal faute de quoi ils seraient une gêne pour le coulissement de la pièce d'écartement lors du mouvement vertical de celle-ci.

L'agrafe 1 de compression est dans un matériau à mémoire de forme mais elle peut avantageusement être également dans un matériau biodégradable et/ou résorbable, stérilisable, d'origine naturelle ou synthétique. Dans le cadre de l'ostéosynthèse, cela permet d'obtenir une consolidation osseuse progressive avec élimination de l'agrafe dans un délai plus ou mois long en fonction de la nature du matériau choisi.

Il peut s'agir d'un matériau choisi dans le groupe :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

Par ailleurs, de préférence lorsque l'agrafe est dans un matériau biodégradable et/ou résorbable, ledit matériau comporte une charge, de préférence à raison de 5 à 60%, d'une poudre, éventuellement d'une nano poudre, ladite charge étant choisie dans le groupe suivant : hydroxy apatite et/ou de phosphate de calcium, notamment de β tricalcium phosphate, et/ou d'hydrogéno phosphate de calcium et/ou de carbonate de calcium et/ou d'hydrogéno carbonate de calcium, de préférence à raison de 5 à 60 % en poids de charge.

La présence de charge permet d'obtenir une reconstruction osseuse plus homogène lors de l'élimination progressive de l'agrafe.

Il est ainsi possible d'obtenir, dans un délai de six mois, une perte de l'ordre de ou inférieure à 50% des propriétés mécaniques initiales de l'agrafe, cette perte étant de 100 % dans un délai d'environ un an.

## Revendications

1. Ensemble chirurgical de pose d'agrafe de compression comprenant :
- une agrafe (1) comprenant deux pattes longitudinales (2), à implanter dans l'os, reliées par un pont transversal (3),
- et un ancillaire de pose, **caractérisé en ce que** :
l'agrafe (1) est dans un matériau à mémoire de forme, ayant au repos ses pattes (2) qui sont convergentes d'un angle α et, en configuration active d'implantation dans l'os, ses pattes soit qui sont parallèles, soit qui ont un angle de convergence β inférieur à α,
et **en ce que** l'ancillaire comporte :
a) une ouverture distale débouchant sur une cavité interne (9) qui sert de logement pour l'agrafe au repos, ses pattes étant tournées vers ladite ouverture et
b) une pièce d'écartement (16), actionnable en déplacement selon un premier mouvement vertical dans lequel la pièce d'écartement (16) vient en contact avec les faces internes des deux pattes (2) et applique sur celles-ci une force réalisant leur écartement jusqu'à la configuration active d'implantation et
c) des moyens d'entraînement de l'agrafe (1) en configuration active, incluant la pièce d'écartement (16), selon un mouvement horizontal permettant la sortie de l'agrafe (2) hors de son logement (9) par l'ouverture distale (8).

2. Ensemble chirurgical selon la revendication 1 **caractérisé en ce que** la pièce d'écartement (16) est une plaque dont l'extrémité a une forme générale de trapèze isocèle (17), la petite base (17a) dudit trapèze (17) correspondant au côté d'introduction de la pièce d'écartement (16) dans l'espace interne entre les deux pattes (2) de l'agrafe (1) au repos, en avant du pont transversal (3) et **en ce que** les faces latérales divergentes (17b) dudit trapèze isocèle (17) sont conformées en sorte de s'appliquer sur les faces internes des deux pattes (2) de l'agrafe (1) et de repousser celles-ci, lors du premier mouvement vertical, jusqu'à ce que les dites pattes (2) soient dans la configuration active d'implantation dans l'os.

3. Ensemble chirurgical selon la revendication 1 **caractérisé en ce que** les moyens d'entraînement consistent en un poussoir (14), à l'avant et sous lequel est montée la pièce d'écartement (16), ledit poussoir (14) comportant une tête supérieure de manoeuvre (15), une tête inférieure (18), une tige verticale de liaison (19) entre les deux dites têtes, **en ce que** l'ancillaire (5) comporte au droit de la cavité interne (9), servant de logement à l'agrafe (1), une première ouverture (10) débouchant sur sa face supérieure et une seconde ouverture (11) débouchant sur sa face inférieure, et **en ce que** le poussoir (14) est monté sur l'ancillaire (5) avec la tige de liaison (19) qui passe à travers la cavité interne (9) et les deux dites ouvertures (10,11) et avec les têtes supérieure (15) et inférieure (18) qui font office de butées respectivement contre la face supérieure et contre la face inférieure de l'ancillaire (5), de part et d'autre desdites ouvertures (10,11), lors du mouvement horizontal du poussoir (14).

4. Ensemble chirurgical selon la revendication 3 **caractérisé en ce que** la partie distale (5a) de l'ancillaire (5) est composée de deux bras latéraux (12) globalement parallèles, s'étendant de part et d'autre de la cavité interne (9) et des deux ouvertures (10,11) supérieure et inférieure et de deux montants transversaux (13) s'étendant entre les deux fils bras latéraux (12) de part et d'autre de l'ouverture distale (8).

5. Ensemble chirurgical selon la revendication 4 **caractérisé en ce que** dans sa position au repos de l'agrafe (1) à l'intérieur de la cavité interne (9) les extrémités libres des deux pattes latérales (2) reposent sur les deux montants transversaux (13) s'étendant entre les deux bras latéraux (12) de part et d'autre de l'ouverture distale (8).

6. Ensemble chirurgical selon la revendication 3 **caractérisé en ce que** la partie distale (25a) de l'ancillaire (25) est délimitée par deux bras latéraux flexibles (32), s'étendant de part et d'autre de la cavité interne (29) et des deux ouvertures supérieure (30) et inférieure (31), les deux dits bras (32) étant convergents au repos et étant aptes à s'écarter l'un de l'autre jusqu'à devenir sensiblement parallèles lors du mouvement horizontal du poussoir (34) du fait qu'ils sont repoussés par la pièce d'écartement (36) et éventuellement la tige de liaison (39).

7. Ensemble chirurgical selon la revendication 6 **caractérisé en ce que** les deux pattes (2) convergentes au repos de l'agrafe (1) sont en appui dans la partie (40) de cavité interne (39) qui est formée dans les deux bras latéraux flexibles (32) convergents.

8. Ensemble chirurgical selon l'une des revendications 6 ou 7 **caractérisé en ce que** la partie distale de l'ancillaire (25) est exempte de montants transversaux de sorte que le poussoir (34) peut, en fin du mouvement horizontal, se détacher de l'ancillaire (25) avec l'agrafe (1) en configuration active d'implantation dans l'os.

9. Ensemble chirurgical selon l'une des revendications 1 à 8 **caractérisé en ce que** l'ancillaire (5) comporte vers sa partie proximale (5b) deux guides tubulaires (6) de perçage, dont l'entraxe correspond à l'écartement des pattes latérales (2) de l'agrafe (1) en position active et, éventuellement, un foret (7) de perçage dont le diamètre correspond à celui desdites pattes (2).

10. Ensemble chirurgical selon l'une des revendications 1 à 9 **caractérisé en ce que** l'extrémité proximale (5b) de l'ancillaire (5) comporte une rainure transversale (23), configurée pour recevoir de manière sensiblement ajustée le pont (3) de l'agrafe (1), après implantation de celle-ci dans l'os (22), en vue de finaliser son impaction.

11. Ensemble chirurgical selon l'une des revendications 1 à 10 **caractérisé en ce que** l'agrafe (1) comporte des ergots (43), anti-migration, sur les faces internes des deux pattes latérales (2), exception faite de la zone d'application de la pièce d'écartement (16), à proximité du pont transversal (3).

12. Ensemble chirurgical selon l'une des revendications 1 à 11 **caractérisé en ce que** l'agrafe (1) est dans au moins un matériau biodégradable et/ou résorbable, stérilisable, d'origine naturelle ou synthétique, notamment choisi dans le groupe :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

13. Ensemble chirurgical selon la revendication 12 **caractérisé en ce que** le matériau dans lequel est réalisée l'agrafe est chargé de poudre et/ou de nano poudre d'hydroxy-apatite et/ou de phosphate de calcium, notamment de β tricalcium phosphate, et/ou d'hydrogéno phosphate de calcium et/ou de carbonate de calcium et/ou d'hydrogéno carbonate de calcium, de préférence à raison de 5 à 60% en poids de charge.

## Claims

1. A surgical assembly for the fitting of compression staples including:
- a staple (1) with two longitudinal pins (2), to be implanted into the bone, connected together by a transverse bridge (3),
- and a fitting ancillary, **characterised in that**:
the staple (1) is made from a shape-memory material, which at rest has its pins (2) converging at an angle α, and in its active configuration of implantation in the bone has its pins either parallel or at an angle of convergence β which is less than α,
and **in that** the ancillary includes:
a) a distal opening that opens into an internal cavity (9) which acts as a housing for the staple at rest, with its pins being turned toward the said opening and
b) a distance piece (16), capable of being moved in a first vertical movement in which the distance piece (16) comes into contact with the inner faces of the two pins (2), and applies to the latter a force that moves them apart to the active implanting configuration and
c) means with which to drive the staple (1) in the active configuration, including the distance piece (16), in a horizontal movement that is used to extract the staple (2) out of its housing (9) via the distal opening (8).

2. A surgical assembly according to claim 1,
**characterised in that** the distance piece (16) is a plate whose end has the general shape of an isosceles trapezium (17), with the small base (17a) of the said trapezium (17) corresponding to the insertion side of the distance piece (16) into the internal space between the two pins (2) of the staple (1) at rest, in front of the transverse bridge (3), and **in that** the diverging lateral faces(17b) of the said isosceles trapezium (17) are shaped so as to bear against the inner faces of the two pins (2) of the staple (1) and push the latter back, during the first vertical movement, until the said pins (2) are in the active configuration of implantation into the bone.

3. A surgical assembly according to claim 1,
**characterised in that** the drive means consist of a push control (14), in front of and below which is mounted the distance piece (16), with the said push control (14) that includes a top control head (15), a bottom head (18), a vertical connecting rod (19) between the said two heads, **in that** the ancillary (5) includes, at the level of the internal cavity (9) which is used as a housing for the staple (1), a first opening (10) that opens out onto on its upper face and a second opening (11) that opens out onto on its lower face, and **in that** the push control (14) is mounted on the ancillary (5) with the connecting rod (19) that passes through the-internal cavity (9) and the two said openings (10,11), and with the top (15) and bottom (18) heads that respectively act as end-stops against the upper face and against the lower face of the ancillary (5), on either side of the said openings (10,11)., during the horizontal movement of the push control (14).

4. A surgical assembly according to claim 3,
**characterised in that** the distal part (5a) of the ancillary (5) is composed of two generally parallel lateral arms (12), lying on either side of the internal cavity (9), of the two upper and lower openings (10,11), and of two transverse elements (13) extending between the two lateral arms (12) on either side of the distal opening (8).

5. A surgical assembly according to claim 4,
**characterised in that**, in the rest position of the staple (1) inside the internal cavity (9), the free ends of the two lateral pins (2) rest on the two transverse elements (13) extending between the two lateral arms (12) on either side of the distal opening (8).

6. A surgical assembly according to claim 3,
**characterised in that** the distal part (25a) of the ancillary (25) is outlined by two flexible lateral arms (32), extending on either side of the internal cavity (29) and of the two upper (30) and lower (31) openings, with the said two arms (32) being convergent at rest, and being designed to move away from each other until they are more-or-less parallel during the horizontal movement of the push control (34) due to being pushed back by the distance piece (36) and possibly the connecting rod (39).

7. A surgical assembly according to claim 6,
**characterised in that** the two convergent pins (2) in the rest position of the staple (1) are pressed into the part (40) of the internal cavity (39) that is formed in the two converging flexible lateral arms (32).

8. A surgical assembly according to one of claims 6 or 7, **characterised in that** the distal part of the ancillary (25) is free of transverse uprights so that, at the end of the horizontal movement, the push control(34) is able to detach itself from the ancillary (25) with the staple (1) in its active configuration for implantation into the bone.

9. A surgical assembly according to one of claims 1 to 8, **characterised in that**, toward its proximal part (5b), the ancillary (5) includes two tubular drilling guides (6), whose distance between centres corresponds to the separation between the lateral pins (2) of the staple (1) in the active position and possibly a drill bit (7) whose diameter is that of the said pins (2).

10. 1a surgical assembly according to one of claims 1 to 9, **characterised in that** the proximal end (5b) of the ancillary (5) includes a transverse groove (23) that is configured to receive the bridge (3) of the staple (1) in a substantially adjusted manner, after implantation of the latter into the bone (22), with a view to finalising its impaction.

11. A surgical assembly according to one of claims 1 to 10, **characterised in that** the staple (1) includes anti-migration studs (43), on the inner faces of the two lateral pins (2), except for the zone of application of the distance piece (16), close to the transverse bridge (3).

12. A surgical assembly according to one of claims 1 to 11, **characterised in that** the staple (1) is in at least one biodegradable and / or re-absorbable, sterilisable material, of natural or synthetic origin, chosen from the following group in particular:
- polylactide, polyglycolide, poly-ε-caprolactone,
- polyhydroxy butyrate, polyhydroxy valerate,
- poly carbonates and similar,
- cellulose, polysaccharides, starch,
as well as their homopolymers, copolymeres and derivatives.

13. A surgical assembly according to claim 12,
**characterised in that** the material from which the staple is made is loaded with a powder and / or nanopowder of hydroxyapatite and / or calcium phosphate, and in particular of β tricalcium phosphate, and / or calcium dihydrogen phosphate and / or calcium carbonate and / or calcium dihydrogen carbonate, preferably at a content of 5 to 60% by weight.

## Patentansprüche

1. Chirurgisches Kit zum Einsetzen einer Kompressionsklammer, umfassend:
- eine Klammer (1), die zwei Längsglieder (2) umfaßt, die in den Knochen zu implantieren sind und durch einen Quersteg (3) verbunden sind,
- und ein Hilfsinstrument zum Einsetzen,
**dadurch gekennzeichnet, daß**:
die Klammer (1) aus einem Material mit Formgedächtnis hergestellt ist, wobei die Glieder (2) im Ruhezustand in einem Winkel α konvergieren und die Glieder in der aktiven Konfiguration zur Implantation in den Knochen entweder parallel sind oder in einem Winkel β zusammenlaufen, der kleiner als α ist, und
daß das Hilfsinstrument umfaßt:
a) eine distale Öffnung, die in einen inneren Hohlraum (9) mündet, der zur Unterbringung der Klammer im Ruhezustand dient, wobei ihre Glieder zu der Öffnung hingewandt sind, und
b) ein Spreizteil (16), das zur Verschiebung in einer ersten, vertikalen Bewegung betätigbar ist, bei der das Spreizteil (16) zu den Innenflächen der beiden Glieder (2) in Kontakt gerät und auf diese eine Kraft ausübt, die ihre Spreizung bis in die aktive Konfiguration zur Implantation bewirkt, und
c) Mittel zum Mitnehmen der Klammer (1) in der aktiven Konfiguration, einschließlich des Spreizteils (16), in einer horizontalen Bewegung, die den Austritt der Klammer (2) aus ihrer Unterbringung (9) durch die distale Öffnung (8) ermöglicht.

2. Chirurgisches Kit nach Anspruch 1, **dadurch gekennzeichnet, daß** das Spreizteil (16) eine Platte ist, deren Ende die allgemeine Form eines gleichschenkeligen Trapezes (17) aufweist, wobei die kürzere Grundlinie (17a) des Trapezes (17) der Seite der Einführung des Spreizteils (16) in den Innenraum zwischen den zwei Gliedern (2) der Klammer (1) im Ruhezustand vor dem Quersteg (3) entspricht, und daß die auseinanderlaufenden Seitenflächen (17b) des gleichschenkeligen Trapezes (17) derart ausgestaltet sind, daß sie sich an die Innenflächen der beiden Glieder (2) der Klammer (1) anlegen und diese im Zuge der ersten, vertikalen Bewegung auseinanderdrücken, bis die Glieder (2) in der aktiven Konfiguration zur Implantation in den Knochen vorliegen.

3. Chirurgisches Kit nach Anspruch 1, **dadurch gekennzeichnet, daß** die Mittel zum Mitnehmen aus einem Druckknopf (14) bestehen, an dem vorne und darunter das Spreizteil (16) angebracht ist, wobei der Druckknopf (14) einen oberen Kopf (15) zur Handhabe, einen unteren Kopf (18) und einen vertikalen Verbindungsstift (19) zwischen den beiden Köpfen umfaßt, daß das Hilfsinstrument (5) an dem inneren Hohlraum (9), der zur Unterbringung der Klammer (1) dient, eine erste Öffnung (10), die sich an seiner Oberseite öffnet, und eine zweite Öffnung (11) aufweist, die sich an seiner Unterseite öffnet, und daß der Druckknopf (14) am Hilfsinstrument (5) angebracht ist, wobei der Verbindungsstift (19) durch den inneren Hohlraum (9) und die beiden Öffnungen (10,11) tritt und der obere (15) und der untere Kopf (18) zu beiden Seiten der Öffnungen (10,11) als Anschläge für die Oberseite bzw. die Unterseite des Hilfsinstruments (5) bei der horizontalen Bewegung des Druckknopfs (14) dienen.

4. Chirurgisches Kit nach Anspruch 3, **dadurch gekennzeichnet, daß** der distale Abschnitt (5a) des Hilfsinstruments (5) aus zwei im allgemeinen parallelen Seitenarmen (12), die sich zu beiden Seiten des inneren Hohlraums (9) und der oberen und der unteren Öffnung (10,11) erstrecken, und zwei Querstreben (13), die sich zu beiden Seiten der distalen Öffnung (8) zwischen den beiden Seitenarmen (12) erstrecken, gebildet ist.

5. Chirurgisches Kit nach Anspruch 4, **dadurch gekennzeichnet, daß** die freien Enden der beiden Seitenglieder (2) im Ruhezustand der Klammer (1) im Inneren des inneren Hohlraums (9) an den beiden Querstreben (13), die sich zu beiden Seiten der distalen Öffnung (8) zwischen den beiden Seitenarmen (12) erstrecken, aufliegen.

6. Chirurgisches Kit nach Anspruch 3, **dadurch gekennzeichnet, daß** der distale Abschnitt (25a) des Hilfsinstruments (25) durch zwei biegsame Seitenarme (32) abgegrenzt ist, die sich zu beiden Seiten des inneren Hohlraums (29) und der oberen (30) und der unteren Öffnung (31) erstrecken, wobei die beiden Arme (32) im Ruhezustand konvergieren und dazu geeignet sind, im Zuge der horizontalen Bewegung des Druckknopfs (34) auseinandergespreizt zu werden, bis sie im wesentlichen parallel sind, da sie vom Spreizteil (36) und gegebenenfalls vom Verbindungsstift (39) auseinandergedrückt werden.

7. Chirurgisches Kit nach Anspruch 6, **dadurch gekennzeichnet, daß** die beiden konvergierenden Glieder (2) im Ruhzustand der Klammer (1) im Abschnitt (40) des Hohlraums (39) aufliegen, der in den beiden konvergierenden, biegsamen Seitenarmen (32) ausgebildet ist.

8. Chirurgisches Kit nach einem der Ansprüche 6 oder 7, **dadurch gekennzeichnet, daß** der distale Abschnitt des Hilfsinstruments (25) keine Querstreben aufweist, so daß sich der Druckknopf (34) am Ende der horizontalen Bewegung mit der Klammer (1) in der aktiven Konfiguration zur Implantation in den Knochen vom Hilfsinstrument (25) lösen kann.

9. Chirurgisches Kit nach einem der Ansprüche 1 bis 8, **dadurch gekennzeichnet, daß** das Hilfsinstrument (5) zu seinem proximalen Abschnitt (5b) hin zwei röhrenförmige Bohrbuchsen (6), deren Achsabstand dem Abstand zwischen den Seitengliedern (2) der Klammer (1) in aktiver Stellung entspricht, und gegebenenfalls ein Bohrwerkzeug (7), dessen Durchmesser jenem der Glieder (2) entspricht, aufweist.

10. Chirurgisches Kit nach einem der Ansprüche 1 bis 9, **dadurch gekennzeichnet, daß** das proximale Ende (5b) des Hilfsinstruments (5) eine Querrille (23) umfaßt, die dazu konfiguriert ist, den Steg (3) der Klammer (1) nach der Implantation selbiger in den Knochen (22) auf im wesentlichen angepaßte Weise aufzunehmen, um ihre Einsetzung zu vervollständigen.

11. Chirurgisches Kit nach einem der Ansprüche 1 bis 10, **dadurch gekennzeichnet, daß** die Klammer (1) an den Innenflächen der beiden Seitenglieder (2) mit Ausnahme des Anlegungsbereichs des Spreizteils (16) in der Nähe des Querstegs (3) bewegungsverhindernde Zapfen (43) aufweist.

12. Chirurgisches Kit nach einem der Ansprüche 1 bis 11, **dadurch gekennzeichnet, daß** die Klammer (1) aus mindestens einem biologisch abbaubaren und/oder resorbierbaren, sterilisierbaren Material natürlichen oder synthetischen Ursprungs hergestellt ist, das insbesondere aus der folgenden Gruppe ausgewählt ist:
- Polylactid, Polyglykolid, Poly-ε-caprolacton,
- Polyhydroxybutyrat, Polyhydroxyvalerat,
- Polycarbonate und ähnliches,
- Cellulose, Polysaccharide, Amidon
sowie deren Homopolymere, deren Copolymere und deren Derivate.

13. Chirurgisches Kit nach Anspruch 12, **dadurch gekennzeichnet, daß** das Material, aus dem die Klammer hergestellt ist, Pulver und/oder Nanopulver von Hydroxylapatit und/oder Calciumphosphat, insbesondere β-Tricalciumphosphat, und/oder Calciumhydrogenphosphat und/oder Calciumcarbonat und/oder Calciumhydrogencarbonat als Füllstoff aufweist, vorzugsweise in einer Menge von 5 bis 60 Gew.-% Füllstoff.
